(19) Europäisches Patentamt
European Patent Office
Office européen des brevets

(11) Veröffentlichungsnummer: **0 405 271 A1**

# (12) EUROPÄISCHE PATENTANMELDUNG

(21) Anmeldenummer: **90111398.5**

(22) Anmeldetag: **16.06.90**

(51) Int. Cl.[5]: **C07D 317/20**, C07D 319/06

(30) Priorität: **29.06.89 DE 3921280**

(43) Veröffentlichungstag der Anmeldung:
**02.01.91 Patentblatt 91/01**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Weber, Jürgen, Dipl.-Chem. Dr.**
**Bunsenstrasse 17**
**D-4200 Oberhausen 11(DE)**
Erfinder: **Lappe, Peter, Dipl.-Chem. Dr.**
**Eickenhof 34**
**D-4220 Dinslaken(DE)**
Erfinder: **Springer, Helmut, Dipl.-Ing.**
**Borbecker Strasse 19**
**D-4200 Oberhausen 11(DE)**

## (54) Verfahren zur Herstellung von Derivaten des 1,3-Dioxolans und des 1,3-Dioxans.

(57) Die Erfindung betrifft ein Verfahren zur Herstellung von Derivaten des 1,3-Dioxolans und des 1,3-Dioxans aus 2,2-Dialkyl-3-hydroxypropanalen in Form des bei der Addition von Formaldehyd an 2-Alkylalkanal unter Einwirkung eines tertiären Amins erhaltenen Rohproduktes und eines Diols.

EP 0 405 271 A1

## VERFAHREN ZUR HERSTELLUNG VON DERIVATEN DES 1,3-DIOXOLANS UND DES 1,3-DIOXANS

Die vorliegende Erfindung betrifft ein neues Verfahren zur Herstellung von 2-[(1,1-Dialkyl-2-hydroxy)-ethyl]-1,3-dioxolanen und 2-[(1,1-Dialkyl-2-hydroxy)ethyl]-1,3-dioxanen der allgemeinen Formel

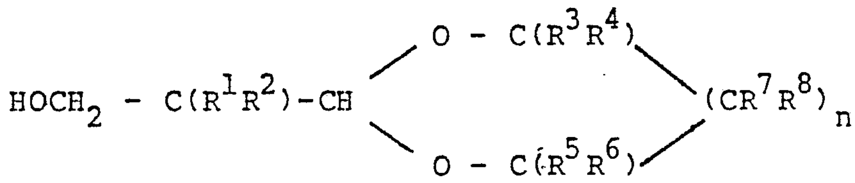

Dioxolane und Dioxane, die der vorstehenden Formel entsprechen, können als cyclische Hydroxyacetale aufgefaßt werden. Dementsprechend ist ein gängiger Weg zu ihrer Gewinnung die Kondensation von Aldehyden mit 1,2- oder 1,3-Diolen. Die substituierten Dioxalane und Dioxane der Erfindung haben große Bedeutung als Zwischenprodukte für die Herstellung von Kunststoffen und Pharmazeutika sowie als Weichmacher für Celluloseacetate als Lackrohstoffe und als Bestandteil von Druckfarben.

Für die Darstellung von Dioxolan- und Dioxanderivaten aus Aldehyden und Diolen sind verschiedene Arbeitsweisen bekannt, die sich durch die Reaktionsbedingungen und insbesondere durch den eingesetzten Katalysator unterscheiden.

So erhält man nach der japanischen Patentanmeldung 16 867 (1966), zitiert in C.A. 66, 10943 d (1967) in 2-Stellung substituierte 5,5-Dimethyl-1,3-dioxane aus 2,2-Dimethylpropan-1,3-diol und einem Aldehyd bei erhöhter Temperatur in Gegenwart eines stark sauren Kationenaustauschers. Setzt man z.B. 2,2-Dimethyl-3-hydroxypropanal ein, dann fällt bei einer Reaktionstemperatur von 50 bis 60 °C 2-[(1,1-Dimethyl-2-hydroxy)-ethyl]-5,5-dimethyl-1,3-dioxan in 64,1 %iger Ausbeute an.

Galiano et al beschreiben in J.Org.Chem. 29, 3424 ff (1964) die Bildung cyclischer Dioxane in Wasser als Reaktionsmedium. Bei Verwendung von 2,2-Dimethylpropan-1,3-diol als Alkohol und 2,2-Dimethyl-3-hydroxypropanal als Aldehyd, die Ausgangsstoffe werden offensichtlich in reiner Form eingesetzt, entsteht bei 75 °C und in Gegenwart von p-Toluolsulfonsäure als Katalysator das entsprechende cyclische Acetal 2-[(1,1-Dimethyl-2-hydroxy)ethyl]-5,5-dimethyl-1,3-dioxan in einer Ausbeute von 88 %. Diese Arbeitsweise ist ungewöhnlich, denn es ist üblich, das sich bei der Umsetzung von Aldehyd und Diol einstellende Gleichgewicht durch azeotrope Abtrennung des Reaktionswassers in Richtung des cyclischen Acetals zu verschieben. Für eine technische Anwendung ist dieser Prozeß nicht geeignet, da er lange Reaktionszeiten erfordert, für die beschriebene Umsetzung z.B. 16 h.

Nach Späth et al, Ber. 76, 949 ff (1943) bringt man 2,2-Dimethylpropan-1,3-diol und 2,2-Dimethyl-3-hydroxypropanal bei 100 °C unter Einwirkung trockenen, gasförmigen Chlorwasserstoffs zur Reaktion. Nach 14 h erhält man das gewünschte Kondensationsprodukt in 56 % Ausbeute. Auch diese Arbeitsweise genügt nicht den Erfordernissen einer technischen Anwendung.

Die Umsetzung von 2,2-Dimethyl-3-hydroxypropanal mit verschiedenen 1,2-Diolen wie Ethylenglykol oder 2,3-Butandiol und 1,3-Diolen wie 1,3-Butandiol oder 1,3-Propandiol zu cyclischen Acetalen ist Gegenstand der DE-OS 19 57 621. Nach diesem Verfahren arbeitet man mit p-Toluolsulfonsäure oder mit Mineralsäure als Katalysator und entfernt das Reaktionswasser destillativ. Die cyclischen Acetale werden in einer Ausbeute von 63 bis 70 % erhalten.

Das aus 2,2-Dimethylpropan-1,3-diol und 2,2-Dimethyl-3-hydroxypropanal erhältliche cyclische Acetal kann auch unmittelbar aus 2,2-Dimethylpropan-1,3-diol gewonnen werden, indem man das Diol mit einem Pt/Kohlenstoff-Katalysator auf 190 °C erhitzt. Nach 5,5 h Reaktionszeit beträgt die Ausbeute an cyclischem Acetal 41,1 %. Führt man dieselbe Umsetzung in Gegenwart eines Pd/Kohlenstoff-Katalysators durch, so erhält man das cyclische Acetal nach 8 h Reaktionszeit in einer Ausbeute von 26 % (GB-PS 1 046 608).

Wie aus der Schilderung des Standes der Technik ersehen werden kann, weisen die bekannten Verfahren zur Herstellung substituierter Dioxolane und Dioxane noch eine Reihe Nachteile auf. So befriedigen die benötigten Reaktionszeiten oder die erzielten Ausbeuten nicht immer; in einigen Fällen erfüllen beide Kriterien nicht die Anforderungen, die an technische Prozesse gestellt werden. Überdies geht aus den veröffentlichten Angaben in der Regel nicht hervor, welcher Reinheitsgrad für die Einsatzstoffe verlangt wird. Lediglich in der DE-OS 19 57 621 findet sich in Beispiel 9 der Hinweis, daß als Aldehydkomponente der Umsetzung ein Rohprodukt mit einer Reinheit von 79 % verwendet wird. Offensichtlich geht der Einsatz eines solchen verunreinigten Ausgangsmaterials zu Lasten der Ausbeute, die nur 49 % der Theorie beträgt.

In diesem Zusammenhang ist zu berücksichtigen, daß der Aldehyd in reiner Form nur mit großem apparativem Aufwand und in schlechter Ausbeute zu erhalten ist.

Es besteht daher Interesse an einem Prozeß zur Herstellung cyclischer Acetale, der auch bei Einsatz von Ausgangsstoffen technischer Reinheit bei kurzen Reaktionszeiten hohe Ausbeuten sicherstellt.

Die Erfindung besteht in einem Verfahren zur Herstellung von Derivaten des 1,3-Dioxolans und des 1,3-Dioxans der allgemeinen Formel

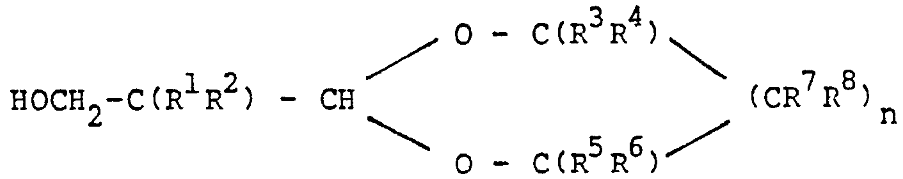

in der $R^1$ und $R^2$ gleich oder verschieden sind und geradkettige oder verzweigte Alkylreste mit 1 bis 4 Kohlenstoffatomen bedeuten, $R^3$, $R^4$, $R^5$, $R^6$, $R^7$, $R^8$ gleich oder verschieden sind und für Wasserstoff oder geradkettige oder verzweigte Alkylreste mit 1 bis 4 Kohlenstoffatome stehen und n O oder 1 ist, durch Kondensation von 2,2-Dialkyl-3-hydroxypropanalen der allgemeinen Formel

$HOCH_2\text{-}CR^1R^2\text{-}CHO$

in der $R^1$ und $R^2$ die obige Bedeutung haben mit Diolen der allgemeinen Formel

$HOCR^3R^4 - (CR^5R^6)_n\text{-} CR^7R^8OH$

in der $R^3$, $R^4$, $R^5$, $R^6$, $R^7$, $R^8$ und n ebenfalls die obige Bedeutung haben. Es ist dadurch gekennzeichnet, daß man die 2,2-Dialkyl-3-hydroxypropanale in Form des bei der Addition von Formaldehyd an 2-Alkylalkanale unter Einwirkung eines tertiären Amins erhaltenen Rohproduktes einsetzt.

Das erfindungsgemäße Verfahren ermöglicht es überraschenderweise, ausgehend von in technischer Reinheit leicht verfügbaren Ausgangsstoffen, substituierte 1,3-Dioxolane und substituierte 1,3-Dioxane bei kurzen Reaktionszeiten in hohen Ausbeuten herzustellen.

Die als eine Komponente der Umsetzung verwendeten 2,2-Dialkyl-3-hydroxypropanale, z.B. 2,2-Dimethyl-3-hydroxypropanal (Formisobutyraldol, Hydroxypivalinaldehyd) werden in bekannter Weise durch Aldoladdition aus Formaldehyd und 2-Alkylalkanalen in Gegenwart von tertiären Aminen erhalten (vgl. z.B. DE-AS 17 93 572). Geeignete 2-Alkylalkanale sind z.B. 2-Methylpropanal, 2-Methylbutanal, 2-Ethylbutanal und 2-Ethylhexanal. Die Reaktion wird in der Regel zwischen 20 und 100 °C, bevorzugt zwischen 60 und 95 °C durchgeführt, wobei man je Mol 2-Alkylalkanal 0,5 bis 1,5 Mol, vorzugweise 1 bis 1,1 Mol Formaldehyd, zweckmäßig als wäßrige Lösung, einsetzt. Die als Katalysatoren verwendeten tertiären Amine gelangen in der Regel in einer Menge von 0,5 bis 25, vorteilhaft von 2 bis 10 Molprozent, bezogen auf das 2-Alkylalkanalen, zum Einsatz. Geeignet sind insbesondere aliphatische tertiäre Amine, wenngleich sich auch cycloaliphatische, araliphatische, aromatische oder heterocyclische Amine als Katalysatoren bewährt haben. Mit Erfolg verwendet werden z.B. Triethylamin, Methyldiethylamin, Tributylamin, Cyclohexyl-dimethylamin, Triethanolamin und insbesondere Tripropylamin.

In der Praxis mischt man 2-Alkylalkanal, Formaldehyd und Amin bei Raumtemperatur und beschleunigt die exotherm verlaufende Reaktion, falls erfoderlich, durch Erhitzen. Anschließend destilliert man aus dem Gemisch Wasser und Amin ab. Das so gewonnene Rohprodukt enthält zwischen 80 und 90 Gew.-% 2,2-Dialkyl-3-hydroxypropanal und zwischen 2 und 4 Gew.-% Amin. Es kann ohne weitere Reinigung dem erfindungsgemäßen Prozeß zugeführt werden.

Die Eignung eines rohen 2,2-Dialkyl-3-hydroxypropanals zur Herstellung cyclischer Acetale in hoher Ausbeute war nicht zu erwarten. Hierbei ist zu berücksichtigen, daß die Rohprodukte neben Amin 10 bis 20 Gew.-% Verunreinigungen enthalten können. Im Falle des aus 2-Methylpropanal (iso-Butyraldehyd) und Formaldehyd gewonnenen 2,2-Dimethyl-3-hydroxypropanals sind das insbesondere Isobutanol, 2,2-Dimethyl-1,3-propandiol und der Neopentylglykolester der Hydroxypivalinsäure sowie nicht umgesetzte Ausgangsaldehyde.

Als Glykole eignen sich u.a. Ethylenglykol, 1,2- und 1,3-Propylenglykol, 1,3-Butandiol, 2,3-Butandiol, 2,2-Dimethyl-1,3-propandiol (Neopentylglykol), 2-Methyl-2-ethyl-1,3-propandiol, 2,2-Diethyl-1,3-propandiol, 2-Ethyl-2-isopropyl-1,3-propandiol, 2,2,4-Trimethyl-1,3-pentandiol, 2-Ethyl-1,3-hexandiol und 2-Ethyl-2-n-butyl-1,3-propandiol. Auch die Glykole können in der handelsüblichen Form eingesetzt werden.

Die Umsetzung der Reaktionspartner kann in an sich bekannter Weise durchgeführt werden. Im allgemeinen wird man sie im Molverhältnis 1 : 1 einsetzen. Es ist selbstverständlich aber auch möglich, entweder den Hydroxyaldehyd oder das Diol im Überschuß zu verwenden. In diesem Fall hat es sich bewährt, je Mol Aldehyd 0,7 bis 1,3, insbesondere 0,8 bis 1,1 Mol des Diols einzusetzen. Die Reaktion

erfolgt in Gegenwart saurer Katalysatoren. Geeignet sind starke Säuren wie Schwefelsäure, Salzsäure, Sulfonsäure und vorzugsweise Phosphorsäure. Die Katalysatormenge, bezogen auf die Ausgangsstoffe, kann sich über einen weiten Bereich erstrecken. Es hat sich bewährt, 0,1 bis 10 Gew.-% und vorzugsweise 0,5 bis 6 Gew.-% Katalysator, bezogen auf den Aldehyd, zu verwenden, wobei dafür Sorge zu tragen ist, daß zuvor das im Reaktionsgemisch enthaltene Amin neutralisiert wurde. Grundsätzlich sind aber auch größere Mengen Mineralsäure anwendbar.

Die Reaktionstemperatur kann in weiten Grenzen variiert werden. Im allgemeinen hält man Temperaturen von 50 bis 180° C, insbesondere 90 bis 140° C ein.

Es ist möglich, die Umsetzung in Gegenwart eines Lösungsmittels durchzuführen. Zweckmäßig dient es gleichzeitig auch als Schleppmittel für das bei der Reaktion entstehende Wasser. Geeignet sind Benzol, Toluol oder Xylol, darüber hinaus auch Chlorkohlenwasserstoffe wie Chloroform, 1,2-Dichloräthan, Tri- oder Perchloräthylen. Die Lösungsmittel werden in Mengen von 20 bis 80 Gew.-%, bezogen auf Aldehyd, eingesetzt.

Unter den beschriebenen Bedingungen erfordert die Umsetzung Reaktionszeiten zwischen 1 und 4 h, insbesondere 1,5 und 3 h.

Zur Aufarbeitung wird das Reaktionsgemisch mit einem alkalisch reagierenden Reagenz, z.B. Natriumhydroxid, Kaliumhydroxid oder Natriumcarbonat in einer solchen Menge versetzt, daß sich ein pH-Wert von etwa 7,0 einstellt. Wasser und organische Phase werden voneinander getrennt, die organische Phase wird fraktioniert destilliert. Nach Abtrennung der leichter flüchtigen Bestandteile des Gemisches, das sind insbesondere organische Lösungsmittel und das Amin sowie niedrigsiedende Nebenprodukte, wird der das Acetal enthaltende Rückstand fraktioniert destilliert. Die Ausbeute beträgt je nach Ausgangsstoffen im allgemeinen mehr als 85 % der Theorie.

In den folgenden Beispielen wird die Erfindung erläutert. Es ist selbstverständlich nicht beabsichtigt, sie auf diese speziellen Ausführungsformen zu beschränken.

<u>Beispiel 1</u>: Herstellung von 2-[(1,1-Dimethyl-2-hydroxy)ethyl]-1,3-dioxan

In einem 2 l-Rundkolben werden 271,0 g 2,2-Dimethyl-3-hydroxypropanal (85,6 %ig, Aminstickstoffgehalt: 0,33 %) (2,27 mol) bei etwa 70° C in 300 g Toluol gelöst. Nach Zugabe von 23,0 g 85 %iger Phosphorsäure (200 mMol) sowie 174,4 g Propandiol-1,3 (96 %ig) (2,20 mol) wird das Gemisch auf Rückflußtemperatur erwärmt und anfallendes Reaktionswasser (49,2 g) über einen Wasserabscheider abgetrennt. Die Reaktionszeit beträgt 2 Stunden. Nach Beendigung der Reaktion läßt man abkühlen und neutralisiert mit 111,8 g 10 %iger Natronlauge bis pH 7,03; es fallen 119,0 g Wasserphase und als organische Phase 712,0 g Rohacetal an, das fraktioniert destilliert wird. Ausbeute: 308,5 g (87,5 % der Theorie).

<u>Beispiel 2</u>: 2-[(1,1-Dimethyl-2-hydroxy)ethyl]-4-methyl-1,3-dioxan

In einem 2 l-Rundkolben werden 271,0 g 2,2-Dimethyl-3-hydroxypropanal (85,6 %ig, Aminstickstoffgehalt: 0,33 %) (2,27 mol) bei etwa 70° C in 300 g Toluol gelöst. Nach Zugabe von 23,0 g 85 %iger Phosphorsäure (200 mMol) und 208,8 g Butandiol-1,3 (94,9 %ig) (2,20 mol) wird das Gemisch auf Rückflußtemperatur erwärmt und anfallendes Reaktionswasser (51,7 g) über einen Wasserabscheider abgetrennt; die Reaktionszeit beträgt 2 Stunden. Nach Beendigung der Reaktion läßt man abkühlen und neutralisiert mit 123,2 g 10 %iger Natronlauge bis pH 7,11; es fallen 127,9 g Wasserphase und als organische Phase 746,4 g Rohacetal an, das fraktioniert destilliert wird. Ausbeute: 337,8 g (88,2 % der Theorie).

<u>Beispiel 3</u>: Herstellung von 2-[(1,1-Dimethyl-2-hydroxy)ethyl]-1,3-dioxolan

In einem 2 l-Rundkolben werden 271,0 g 2,2-Dimethyl-3-hydroxypropanal (85,6 %ig, Aminstickstoffgehalt: 0,33 %) (2,27 mol) bei etwa 70° C in 300 g Toluol gelöst. Nach Zugabe von 23,0 g 85 %iger Phosphorsäure (200 mMol) sowie 136,6 g Ethylenglykol (99,9 %ig) (2,20 mol) wird das Gemisch auf Rückflußtemperatur erwärmt und anfallendes Reaktionswasser (51,4 g) über einen Wasserabscheider abgetrennt; die Reaktionszeit beträgt 2,5 Stunden. Nach Beendigung der Reaktion läßt man abkühlen und neutralisiert mit 132,5 g 10 %iger Natronlauge bis pH 7,10; es fallen 137,9 g Wasserphase und als

organische Phase 673,8 g Rohacetal an, das fraktioniert destilliert wird. Ausbeute: 277,8 g (86,5 % der Theorie).

Beispiel 4: Herstellung von 2-[(1-Methyl-1-ethyl-2-hydroxy)ethyl]-4-methyl-1,3-dioxan

In einem 2 l-Rundkolben werden 258,2 g 2-Hydroxymethyl-2-methylbutanal (89,9 %ig, Aminstickstoffgehalt: 0,21 %) (2,0 mol) bei etwa 70 °C in 300 g Toluol gelöst. Nach Zugabe von 16,1 g 85 %iger Phosphorsäure (140 mMol) und 189,2 g Butandiol-1,3 (94,9 %ig) (2,0 mol) wird das Gemisch auf Rückflußtemperatur erwärmt und anfallendes Reaktionswasser (45,1 g) über einen Wasserabscheider abgetrennt; die Reaktionszeit beträgt 3 Stunden. Nach Beendigung der Reaktion läßt man abkühlen und neutralisiert mit 100,1 g 10 %iger Natronlauge bis pH 7,08; es fallen 109,5 g Wasserphase und als organische Phase 709,0 g Rohacetal an, das fraktioniert destilliert wird. Ausbeute: 321,6 g (85,5 % der Theorie).

Beispiel 5: Herstellung von 2-[(1-Methyl-1-ethyl-2-hydroxy)ethyl]-5,5-dimethyl-1,3-dioxan

In einem 2 l-Rundkolben werden 258,2 g 2-Hydroxymethyl-2-methylbutanal (89,9 %ig, Aminstickstoffgehalt: 0,21 %) (2,0 mol) bei etwa 70 °C in 300 g Toluol gelöst. Nach Zugabe von 16,1 g 85 %iger Phosphorsäure (140 mMol) und 218,4 g Neopentylglykol (99,5 %ig; 2,09 Mol) wird das Gemisch auf Rückflußtemperatur erwärmt und das anfallende Reaktionswasser (41,7 g) über einen Wasserabscheider abgetrennt, die Reaktionszeit beträgt 3 Stunden. Nach Beendigung der Reaktion läßt man abkühlen und neutralisiert mit 72,7 g 10 %iger Natronlauge bis pH 7,11; es fallen 82,8 g Wasserphase als organische Phase und 740,9 g Rohacetal an, das fraktioniert destilliert wird. Ausbeute: 362,3 g (89,7 % der Theorie).

Beispiel 6: Herstellung von 2-[(1,1-Diethyl-2-hydroxy)ethyl]-5,5-dimethyl-1,3-dioxan

In einem 2 l-Rundkolben werden 357,4 g 2-Ethyl-2-hydroxymethylbutanal (80,09 %ig, Aminstickstoffgehalt: 0,44 %) (2,20 mol) bei etwa 70 °C in 300 g Toluol gelöst. Nach Zugabe von 23,1 g 85 %iger Phosphorsäure (200 mMol) und 230,2 g Neopentylglykol (99,52 %ig; 2,20 Mol) wird das Gemisch auf Rückflußtemperatur erwärmt und das anfallende Reaktionswasser (43,8 g) über einen Wasserabscheider abgetrennt, die Reaktionszeit beträgt 3 Stunden. Nach Beendigung der Reaktion läßt man abkühlen und neutralisiert mit 149,6 g 10 %iger Natronlauge bis pH 7,09, es fallen 161,1 g Wasserphase und als organische Phase 855,4 g Rohacetal an, das fraktioniert destilliert wird. Ausbeute: 410,0 g (86,3 % der Theorie).

Beispiel 7: Herstellung von 2-[(1,1-Diethyl-2-hydroxy)ethyl]-1,3-dioxan

In einem 2 l-Rundkolben werden 357,4 g 2-Ethyl-2-hydroxymethylbutanal (80,09 %ig, Aminstickstoffgehalt: 0,44 %) (2,20 mol) bei etwa 70 °C in 300 g Toluol gelöst. Nach Zugabe von 23,1 g 85 %iger Phosphorsäure (200 mMol) und 174,4 g Propandiol-1,3 (96,0 %ig; 2,20 Mol) wird das Gemisch auf Rückflußtemperatur erwärmt und das anfallende Reaktionswasser (54,9 g) über einen Wasserabscheider abgetrennt, die Reaktionszeit beträgt 3 Stunden. Nach Beendigung der Reaktion läßt man abkühlen und neutralisiert mit 163,0 g 10 %iger Natronlauge bis pH 7,15, es fallen 202,8 g Wasserphase und als organische Phase 760,2 g Rohacetal an, das fraktioniert destilliert wird. Ausbeute: 349,5 g (84,5 % der Theorie).

Beispiel 8: Herstellung von 2-[(1,1-Dimethyl-2-hydroxy)ethyl]-5,5-dimethyl-1,3-dioxan

In einem 2 l-Rundkolben werden 392,0 g 2,2-Dimethyl-3-hydroxypropanal (88,7 %ig, Aminstickstoffgehalt: 0,36 %) (3,40 mol) bei etwa 70 °C in 463,6 g Toluol gelöst. Nach Zugabe von 35,6 g 85 %iger Phosphorsäure (309 mMol) und 321,3 g Neopentylglykol (99,52 %ig; 3,08 Mol) wird das Gemisch auf Rückflußtemperatur erwärmt und das anfallende Reaktionswasser (67,2 g) über einen Wasserabscheider abgetrennt, die Reaktionszeit beträgt 2,5 Stunden. Nach Beendigung der Reaktion läßt man abkühlen und

neutralisiert mit 227,2 g 10 %iger Natronlauge bis pH 7,12, es fallen 243,3 g Wasserphase und als organische Phase 1129,2 g Rohacetal, das fraktioniert destilliert wird. Ausbeute: 508,4 g (87,8 % der Theorie).

## Ansprüche

1.) Verfahren zur Herstellung von Derivaten des 1,3-Dioxolans und des 1,3-Dioxans der allgemeinen Formel

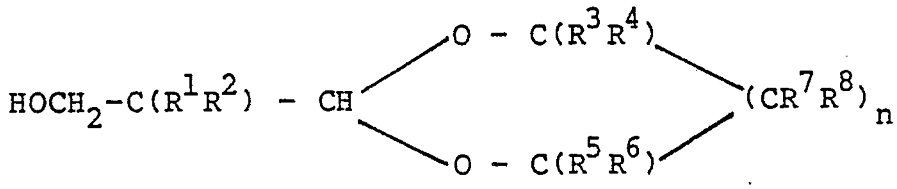

in der $R^1$ und $R^2$ gleich oder verschieden sind und geradkettige oder verzweigte Alkylreste mit 1 bis 4 Kohlenstoffatomen bedeuten, $R^3$, $R^4$, $R^5$, $R^6$, $R^7$, $R^8$ gleich oder verschieden sind und für Wasserstoff oder geradkettige oder verzweigte Alkylreste mit 1 bis 4 Kohlenstoffatome stehen und n O oder 1 ist, durch Kondensation von 2,2-Dialkyl-3-hydroxypropanalen der allgemeinen Formel
$HOCH_2\text{-}CR^1R^2\text{-}CHO$
in der $R^1$ und $R^2$ die obige Bedeutung haben mit Diolen der allgemeinen Formel
$HOCR^3R^4 - (CR^5R^6)_n - CR^7R^8OH$
in der $R^3$, $R^4$, $R^5$, $R^6$, $R^7$, $R^8$ und n ebenfalls die obige Bedeutung haben, dadurch gekennzeichnet, daß man die 2,2-Dialkyl-3-hydroxypropanale in Form des bei der Addition von Formaldehyd an 2-Alkylalkanale unter Einwirkung eines tertiären Amins erhaltenen Rohproduktes einsetzt.

2.) Verfahren nach Anspruch 1, dadurch gekennzeichnet, daß je Mol Aldehyd 0,7 bis 1,3, insbesondere 0,8 bis 1,1 Mol Diol eingesetzt werden.

3.) Verfahren nach Anspruch 1 oder 2, dadurch gekennzeichnet, daß als saurer Katalysator Phosphorsäure eingesetzt wird.

4.) Verfahren nach einem oder mehreren der Ansprüche 1 bis 3, dadurch gekennzeichnet, daß nach Neutralisation des im Reaktionsgemisch enthaltenen Amins 0,1 bis 10 Gew.-%, vorzugsweise 0,5 bis 6 Gew.-% Katalysator, bezogen auf den Aldehyd, eingesetzt werden.

5.) Verfahren nach einem oder mehreren der Ansprüche 1 bis 4, dadurch gekennzeichnet, daß die Reaktion bei Temperaturen von 50 bis 180, insbesondere 90 bis 140 °C durchgeführt wird.

6.) Verfahren nach einem oder mehreren der Ansprüche 1 bis 5, dadurch gekennzeichnet, daß die Reaktion in Gegenwart eines Lösungmittels durchgeführt wird, das gleichzeitig als Schleppmittel für das bei der Reaktion entstehende Wasser dient.

7.) Verfahren nach Anspruch 6, dadurch gekennzeichnet, daß das Schleppmittel ein aromatischer Kohlenwasserstoff oder ein Chlorkohlenwasserstoff ist.

8.) Verfahren nach Anspruch 6 oder 7, dadurch gekennzeichnet, daß das Lösungmittel in einer Menge von 20 bis 80 Gew.-%, bezogen auf Aldehyd, eingesetzt wird.

Europäisches Patentamt

# EUROPÄISCHER RECHERCHENBERICHT

Nummer der Anmeldung

EP 90111398.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | DE - A - 1 957 621 (DYNAMIT NOBEL AG) * Ansprüche; Beispiel 9 * -- | 1-8 | C 07 D 317/20<br>C 07 D 319/06 |
| D,A | GB - A - 1 046 608 (SHELL) * Ansprüche 1,4,15 * -- | 1-8 | |
| D,A | CHEMICAL ABSTRACTS, Band 66, Nr. 3, 16. Jänner 1967 Columbus, Ohio, USA JAPAN GAS-CHEMICAL CO "2--Substituted 5,5-dimthyl-1,3--dioxane" Seite 1067, Spalte 1, Zusammenfassung-Nr. 10 943d & Japan 16 867(66) -- | 1-8 | |
| A | CHEMCIAL ABSTRACTS, Band 103, Nr. 22, 2. Dezember 1985 Columbus, Ohio, USA NIPPON KAYAKU CO "Acrylates" Seite 82, Spalte 1, Zusammenfassung-Nr. 179 746t & Jpn. Kokai Tokkyo Koho JP 6,078,976 (85 79 976) -- | 1 | RECHERCHIERTE SACHGEBIETE (Int Cl 5)<br>C 07 D 317/00<br>C 07 D 319/00 |
| A | CHEMICAL ABSTRACTS, Band 103, Nr. 13, 30. September 1985 Columbus, Ohio, USA NIPPON KAYAKU CO. "Dioxolane bisalkanol di(meth)acrylates" Seite 622, Spalte 1, Zusammenfassung-Nr. 104 950w & Jpn. Kokai Tokkyo Koho JP 6,001,178 (85 01 178) ---- | 1 | |

Der vorliegende Recherchenbericht wurde für alle Patentansprüche erstellt.

| Recherchenort | Abschlußdatum der Recherche | Prüfer |
|---|---|---|
| WIEN | 20-09-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument